# EUROPEAN PATENT APPLICATION

(11) **EP 4 098 273 A1**
(43) Date of publication of application: **07.12.2022**
(21) Application number: 22169956.4
(22) Date of filing: 07.10.2015
(51) Int. Cl.: A61K 38/17, A61K 31/00, A61K 38/16, C07K 14/005, A61K 38/21, A61K 31/522, C07K 14/705, A61K 31/513, A61K 31/675, A61K 31/7072, A61K 45/06, A61P 31/14, A61P 31/20

(54) **COMBINATION THERAPY OF HBV AND HDV INFECTION**

(30) Priority: 07.10.2014 EP 14187865
(62) Divisional of application: 15775453.2
(71) Applicant: Myr GmbH, 30938 Burgwedel (DE)
(72) Inventor: ALEXANDROV, Alexander, 61348 Bad Homburg (DE)
(74) Representative: Carpmaels & Ransford LLP

(57) **Abstract**

The invention provides a composition comprising an inhibitor of Na+-taurocholate cotransporting polypeptide (NTCP) and a active ingredient selected from the group consisting of a nucleoside analogue such as lamivudine, telbivudine, or entecavir, a nucleotide analogue such as tenofovir, adefovir and an immunomodulator such as interferon alpha. The NTCP inhibitor inhibits HBV/HDV entry into a cell and is preferably derived from an HBV pre-Sl peptide. Also provided are methods of treating HBV and HDV infection, hepatitis B and D, or chronic hepatitis B and D.

## Description

### FIELD OF INVENTION

The present invention pertains to novel compositions for the treatment of infections caused by hepatitis B virus (HBV) and hepatitis D virus (HDV), in particular hepatitis B and D. The compositions comprise a combination of an agent acting as an HBV/HDV entry inhibitor, and a nucleotide/nucleoside analogue (NUC) or immunomodulator such as interferon.

### BACKGROUND OF THE INVENTION

### Epidemiology and medical problem related to Hepatitis B virus

Hepatitis B is a potentially life-threatening liver infection caused by the HBV. Worldwide, an estimated two billion people were HBV infected, whereas more than 350 million of those have chronic liver infections (WHO Fact Sheet, 2008).

Chronic hepatitis B (CHB) is a serious global public health issue. Up to 1.2 million deaths per year are caused by HBV-related chronic liver diseases resulting in it being the 10th leading cause of death worldwide (Lavanchy, 2004). Without appropriate treatment, 15-25% of the chronically infected will die prematurely because of the severe and fatal complications of chronic liver disease (CDC Fact Sheet, 2006). Actually, only a relatively small proportion of affected patients receive medication.

### Epidemiology and medical problem related to Hepatitis D virus

About 5% of chronically HBV infected are co-infected with HDV (Taylor, 2006). HDV is a small RNA virus, which requires helper functions from HBV for virion assembly and propagation, and uses the HBV envelope for virus release and infection of new cells. Immunization against HBV also protects against HDV infection. The presence of HDV is associated with more severe and rapid progression of liver disease than HBV infection alone. Liver cirrhosis and cancer occur 10-15 years earlier in HBV/HDV co-infection, and the 5-year mortality of co-infected individuals is twice that of HBV mono-infection (German Guideline for Prophylaxis, Diagnostics and Treatment of HBV infection, 2007).

The therapeutic options for HDV co-infected patients are very limited. Only interferons show some degree of efficacy in a small proportion of patients with approximately 25% of virological and biochemical response. Antiviral agents active against HBV do not work against HDV (Taylor, 2006).

### Treatment goals

Several national guidelines for treatment of HBV infection exist, and the European Association for the Study of the Liver (EASL) clinical practice guideline has recently been published (Journal of Hepatology, 2009). According to this guideline, the goal of therapy for hepatitis B is to improve quality of life and survival by preventing progression of the disease to cirrhosis, end-stage liver disease, HCC and death. The ideal endpoint of the therapy is sustained Hepatitis B virus s antigen (HBsAg) loss with or without seroconversion to anti-HBs. This is associated with a complete and definitive remission of the activity of CHB and an improved long-term outcome. However, this goal can be achieved only in minority of patients with available therapies. Therefore, for the Hepatitis B virus e antigen (HBeAg) positive patients, a durable HBeAg seroconversion and in HBeAg negative patients or HBeAg positive patients who are not able to achieve HBeAg seroconversion, a maintained undetectable DNA level is the next most desirable goal.

### HBV virus

Hepatitis B viruses (HBVs) are small, enveloped DNA viruses that replicate their genome via reverse transcription of a pregenomic RNA transcript in the cytoplasm of infected hepatocytes. They are classified into the family *Hepadnaviridae* and are adapted to mammals (primates and rodents) and birds, where they cause acute and persistent infections. From studies in a HBV-susseptable cell line HepaRG and systems based on primary human hepatocytes (PHH) and primary *Tupaia belangeri* hepatocytes it became clear that viral infectivity can be assigned to different subdomains in two of the three viral envelope proteins. The three HBV envelope proteins are termed L (large), M (middle) and S (small). They form a proteinaceous outer virus shell, which is embedded in an endoplasmic reticulum (ER)-derived lipid bilayer. Their coding mRNAs originate from one single open reading frame. Since their three start codons are in phase, they share the C-terminal 226 amino acids long S-domain which anchor the proteins via four putative transmembrane helices (TM domains) in the lipid bilayer. The S protein drives particle formation and accordingly serves an important function in virus assembly. The S domain has further been shown to participate in virus entry. Linked to the N terminus of the S domain, the M and L proteins bear two hydrophilic extensions, one of 55 amino acids and, depending on genotype, one of 108 (genotype D), 118 (genotypes E and G), or 119 (genotypes A, B, C, F, H) amino acids called pre-S2 (M) and pre-S1 (L), respectively. The L proteins of all hepadnaviruses contain recognition motifs for *N*-myristoyltransferase and are accordingly subjected to this modification. The lack of a classical secretion signal in L results in an initial cytoplasmic orientation of its pre-S domains. After synthesis, myristoylation, and incorporation of the TM helices into the ER membrane, the pre-S domain of L traverses the lipid bilayer, allowing parts of it to become accessible at the particle exterior. In virions the stoichiometric ratio of L, M and S proteins is about 1:1:4, while the more abundantly secreted non-infectious subviral particles (SVPs) contain almost exclusively S protein and only traces of L protein.

Crucial elements for virus attachment, specific receptor binding, or fusion have been identified within the L, M, and S proteins. Myristoylation of L was found to be mandatory for virus infectivity (6, 16). However, the requirement of the N-terminal myristic acid moiety in the inhibitory activity is to some extent variable. Replacement of myristic acid by other hydrophobic saturated and unsaturated fatty acid moieties or even by cholesterol allows the modulation of specific activities according to the relative hydrophobicity. Thus, lipophilicity *per se* and not a specific lipid structure seems to play the key role for this part of the molecule.

Successive deletions of 5 amino acids within the N-terminal pre-S1 sequence of residues 1 to 78 abrogated HBV infectivity. In contrast, most parts of the pre-S2 domain were dispensable for infection. Using HDV, these results were confirmed, indicating that both viruses share very similar entry modes. It was further found that the S domain, either as part of the HBV L protein or as the S protein, being the major constituent of the viral envelope, is required for infectivity. Two separable and distinct functions were identified within the pre-S1 infectivity determinant consisting of residues 1 to 78: one is related to the inhibitory activity of the peptide through interference with a hepatocyte-specific receptor, and the other, involving amino acids 49 to 78, is still unknown.

### Current HBV therapies

Approved drugs for treatment of chronic HBV-infections belong to two classes: antivirals, and immunomodulators such as interferons.

All HBV antivirals currently available belong to the class of nucleoside/nucleotide analogues (NUCs) and act as polymerase inhibitors. More recently approved entecavir and tenofovir are considered to be superior to other approved agents (lamivudine, adefovir and telbuvudine) because of higher potency and higher genetic barrier for resistance in treatment naive patients. All approved anti-HBV drugs show good safety profiles so far.

All of these products block virus replication and lead to a rapid decrease of plasma viral DNA. Patients may also benefit from improvements in liver histology. However, HBsAg seroconversion, the ultimate goal of HBV treatment, is achieved in only 4% of tenofovir patients after two years and is even lower for the other drugs (German Guideline for Prophylaxis, Diagnostics and Treatment of HBV infection, 2007). Furthermore, 70% of patients treated with lamivudine develop resistance against the drug after five years of treatment. For adefovir, the resistance development is slower with 18% of the patients showing resistance after four years. For entecavir this figure is 1.5% in 6 years therapy in naive patients. Nevertheless, a significant population of patients resistant to nucleoside / nucleotide analogues will accumulate over the next years, especially as patients previously treated with lamivudine develop high levels of resistance to entecavir. Reverse transcriptase inhibitors do not prevent the establishment of cccDNA (covalently closed circular DNA) in naive hepatocytes.

Interferon-alpha (INFa) is an immunomodulator with a complex and not completely understood mode of action, involving activation of the immune system to eliminate the infected hepatocytes. Several formulations are marketed with a usual dose of 5 to 10 mg units two or three times weekly. Pegylated forms with delayed release from subcutaneous depots are also available, allowing more patient friendly application only once a week. All interferon treatments are administered by subcutaneous self-injection. The major drawback of interferon treatment for chronic hepatitis B is the combination of moderate efficacy (after 48 weeks of treatment, overall HBsAg seroconversion rates of approximately 3-6%, undetectable HBV DNA in about 20%, depending on the HBV genotype: Lau et al., 2005; Fink et al., 2006) and significant side effects. These may include the cytokine syndrome, severe depression and suicide and are experienced by a substantial part of the patient population. Interferons are the only treatment option showing some therapeutic effects in HBV/HDV co-infection. However, the efficacy in this population is rather limited with only 25% showing sustained virological and biochemical response (Interview with Dr. H. Wedemeyer, Hanover Medical School, Germany).

Thus, current therapies for chronic hepatitis B rarely induce serological cure and thus long-term treatment with HBV polymerase inhibitors is usually required. There is no effective treatment for the majority of hepatitis delta patients.

### HBV/HDV entry inhibitors

A novel class of anti-HBV and HDV molecules, virus entry inhibitors, has recently been described. Its postulated mechanism of antiviral action is the highly specific, highly stable binding to Na+-taurocholate cotransporting polypeptide (NTCP), a sodium/bile acid cotransporter (also known as liver bile acid transporter (LBAT)) located at the basolateral membrane of differentiated hepatocytes. NTCP binding results in abrogation of a productive fusion of the viral and the cellular membranes and thereby prevents an infection of the cell. This unique mechanism of action allows to address two most important medical needs, namely long-term HBV eradication as well as antiviral activity against hepatitis D (or, delta) virus (HDV).

Virus entry inhibitors are derived from the N-terminal domain of the large (L) HBV surface protein, pre-S1. An example of an HBV/HDV entry inhibitor is a linear 47-amino acid chemically synthesized peptide (known under the trade name Myrcludex B) which is derived from the pre-S1 domain of the large (L) HBV surface protein, composed of naturally occurring L-amino acids and bearing an N-terminal myristoyl moiety and a C-terminal carboxamide.

### Combination therapies

Across the medical community, there is a great interest in combination therapies aiming at HBsAg loss after a limited treatment period. However, the results of combination trials using interferons together with nucleoside/nucleotides in HBV and HDV infection were so far disappointing. Thus, combining pegylated Interferon-alpha with a nucleoside analogue lamivudine did not produce an increase in efficacy (Lau et al., 2005).

Taken together, there exists an unmet need for further therapies of HBV and HDV infections and resulting diseases. In particular, such therapies should aim at HBsAg loss, with or without seroconversion to anti-HBs antibodies, a result closest to complete cure.

### SUMMARY OF THE INVENTION

The invention is based on the finding that a combination of an HBV/HDV pre-S1 entry inhibitor with a nucleotide/nucleoside analogue or an immunomudulator such as interferon results in a decrease or loss of HDV RNA in hepatitis D and/or HBsAg in hepatitis B and D. The invention thus provides a novel combination therapy of HBV/HDV infection, hepatitis B or D, or chronic hepatitis B or D.

In one aspect, it is provided a combination or composition comprising an inhibitor of Na+-taurocholate cotransporting polypeptide (NTCP) and a further active ingredient selected from the group consisting of a nucleotide analogue, a nucleoside analogue and an immunomodulator. The combination or composition may comprise more than one further active ingredient. The composition may further comprise a pharmaceutically acceptable excipient, carrier etc.

The NTCP inhibitor may be a small molecule or a pre-S1 peptide inhibitor, wherein the pre-S1 peptide inhibitor comprises a pre-S1 peptide of an HBV virus, or a functional fragment thereof. The functionality of the fragment may be assessed, e.g., based on its ability to bind to NTCP, inhibit NTCP, or inhibit HBV/HDV cell entry. A preferred NTCP inhibitor is a pre-S1 peptide inhibitor.

The HBV virus may be any HBV virus, e.g., HBV strain alpha1, HBV strain LSH, woodchuck HBV, Woolly Monkey HBV (WMHBV), HBV subtype *AD, ADR, ADW, ADYW, AR* or *AYW,* or HBV genotype A to H.

The pre-S1 peptide inhibitor may comprise at least amino acids 2 to 9 and/or 11 to 15 of a pre-S1 peptide of an HBV virus. Alternatively, the pre-S1 peptide inhibitor may comprise at least amino acids 9 to 15 or 11 to 15 of a pre-S1 peptide of an HBV virus. The pre-S1 peptide inhibitor may comprise or consist of amino acids 2 to 48 or 2 to 21 of a pre-S1 peptide of an HBV virus.

Exemplary pre-S1 peptide inhibitor comprises the amino acid sequence
between positions 2 and 48 of the HBV pre-S1 consensus sequence:
   GTNL SVPNP LGFFP DHQLD PAFRA NSNNP DWDFN PNKDH WPEAN KVG (SEQ ID NO: 12), or
between positions 2 and 48 of the HBV pre-S1 Genotype C sequence:
   GTNL SVPNP LGFFP DHQLD PAFGA NSNNP DWDFN PNKDH WPEAN QVG (SEQ ID NO: 14), or
between positions 2 and 48 of the HBV pre-S1 Genotype C sequence with an amino acid substitution at position 46 (Gln (Q) --> Lys (K)):
   GTNL SVPNP LGFFP DHQLD PAFGA NSNNP DWDFN PNKDH WPEAN KVG (SEQ ID NO: 13), or
between positions 2 and 48 of the HBV pre-S1 Genotype D sequence:
   GQNL STSNP LGFFP DHQLD PAFRA NTANP DWDFN PNKDT WPDAN KVG (SEQ ID NO: 5).

The pre-S1 peptide inhibitor may be modified by a hydrophobic moiety at the N-terminus. The hydrophobic moiety modification may be by acylation, e.g., acylation with myristoyl or stearoyl.

A preferred pre-S1 peptide inhibitor has the amino acid sequence of
GTNL SVPNP LGFFP DHQLD PAFGA NSNNP DWDFN PNKDH WPEAN KVG (SEQ ID NO: 13),
wherein the peptide is modified at the N-terminus by myristoyl.

A preferred pre-S1 peptide inhibitor is Myrcludex B having the following chemical formula: N- Myristoyl-glycyl-L-threonyl-L-asparaginyl-L-leucyl-L-seryl-L-valyl-L-prolyl-L-asparaginyl-L-prolyl-L-leucyl-glycyl-L-phenylalanyl-L-phenylalanyl-L-prolyl-L-aspartyl-L-histidyl-L-glutaminyl-L-leucyl-L-aspartyl-L-prolyl-L-alanyl-L-phenylalanyl-glycyl-L-alanyl-L-asparaginyl-L-seryl-L-asparaginyl-L-asparaginyl-L-prolyl-L-aspartyl-L-tryptophanyl-L-aspartyl-L-phenylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamide, acetate salt.

The pre-S1 peptide inhibitor may be modified at the C-terminus or elsewhere to protect the peptide form degradation. Exemplary moieties that can be used for this purpose are D-amino acids, cyclic amino acids, modified amino acids, natural or synthetic polymers such as polyethylene glycol (PEG).

Hereinafter the above NTCP inhibitors, including pre-S1 peptide inhibitors may be referred to as an inhibitor. This designation includes all NTCP inhibitors, preferably pre-S1 peptide inhibitors, of the invention.

The immunomodulator may be a therapeutic vaccine, an adjuvant or an interferon such as interferon alpha (IFNa), e.g., interferon alpha 2a or interferon alpha 2b. The interferon in the composition may be pelylated (PEG-IFN).

The nucleoside analogues may be lamivudine, telbivudine or entecavir.

The nucleotide analogues may be tenofovir or adefovir.

Hereinafter the above further active ingredients may be referred to as a further active ingredient or a further active agent. This designation includes all nucleoside analogues, nucleotide analogues and immunomodulators, of the invention.

Two or more of the above further active ingredients may be used in the combination or composition of the invention.

Accordingly, provided are *inter alia* combinations and compositions comprising following ingredients:
a pre-S1 peptide inhibitor such as Myrcludex B and IFNa or PEG-IFNa;
a pre-S1 peptide inhibitor such as Myrcludex B and lamivudine;
a pre-S1 peptide inhibitor such as Myrcludex B and telbivudine;
a pre-S1 peptide inhibitor such as Myrcludex B and entecavir;
a pre-S1 peptide inhibitor such as Myrcludex B and tenofovir; or
a pre-S1 peptide inhibitor such as Myrcludex B and adefovir.

A unit dose of Myrcludex B in the combination or composition may be between 0.5 mg an 20 mg, for example 1 mg, 2 mg, 3 mg, 4, mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, preferably 2 mg, 5 mg, or 10 mg.

A unite dose of interferon or pegylated interferon in the combination or composition may be between 10 µg and 300 µg, for example, 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg, preferably 180 µg.

A unit dose of lamivudine in the combination or composition may be between 10 mg and 100 mg, for example 10 mg, 20 mg, 30 mg, 40 mg, 50 mg, 60 mg, 70 mg, 80 mg, 90 mg or 100 mg, preferably 100 mg.

A unit dose of entecavir in the combination or composition may be between 0.1 mg and 10 mg, for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, 10.0 mg, preferably 0.5 mg or 1.0 mg.

A unit dose of telbivudine in the combination or composition may be between 100 mg and 1000 mg, for example 100 mg, 200 mg, 300 mg, 400 mg, 500 mg, 600 mg, 700 mg, 800 mg, 900 mg, or 1000 mg, preferably 500 mg, 600 mg, or 700 mg.

A unite dose of tenofovir in the combination or composition may be between 100 mg and 1000 mg, for example 100 mg, 200 mg, 300 mg, 400 mg, 500 mg, 600 mg, 700 mg, 800 mg, 900 mg, or 1000 mg, preferably 200 mg, 250 mg, 245 mg, or 300 mg.

A unite dose of adefovir in the combination or composition may be between 5 mg to 20 mg, for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, preferably 10 mg.

In another aspect, the invention provides a combination or a composition as defined above for use in a method of treatment of HBV or HDV infection in a subject, hepatitis B, chronic hepatitis B, hepatitis D, or chronic hepatitis D.

In another aspect, the invention provides a pre-S1 peptide inhibitor for use a method of treatment of HBV or HDV infection, hepatitis B, chronic hepatitis B, hepatitis D, or chronic hepatitis D in a subject, wherein the method comprises the administration of a further active ingredient, wherein the further active ingredient is selected from a group consisting of an immunomodulator, preferably interferon; a nucleotide analogue, preferably tenofovir or adefovir; and an nucleoside analogue, preferably lamivudine, telbivudine and entecavir.

In another aspect, the invention provides a method of treatment of HBV or HDV infection, hepatitis B, chronic hepatitis B, hepatitis D, or chronic hepatitis D in a subject, comprising administering to the subject an inhibitor of Na+-taurocholate cotransporting polypeptide (NTCP), preferably a pre-S1 peptide inhibitor, and a further active ingredient selected from the group consisting of a nucleotide analogue, a nucleoside analogue and an immunomodulator.

Said inhibitor and said further active ingredient may be provided as a combination or a composition as defined above. More than one further active ingredient may be used in the methods.

Thus, the methods may, for example, comprise administering:
a pre-S1 peptide inhibitor such as Myrcludex B and IFNa or PEG-IFNa;
a pre-S1 peptide inhibitor such as Myrcludex B and lamivudine;
a pre-S1 peptide inhibitor such as Myrcludex B and telbivudine;
a pre-S1 peptide inhibitor such as Myrcludex B and entecavir;
a pre-S1 peptide inhibitor such as Myrcludex B and tenofovir; or
a pre-S1 peptide inhibitor such as Myrcludex B and adefovir.

The method may comprise:
administering a pre-S1 peptide inhibitor in a dose of between 0.5 an 20 mg per day, for example 1 mg, 2 mg, 3 mg, 4, mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg per day, preferably 2 mg, 5 mg or 10 mg per day for HDV and HBV infection,
and further administering of one or more of following further active ingredients:
   administering pegylated interferon in the dose of between 10 µg and 300 µg per week, for example, 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg per week, preferably 180 µg per week. A weekly dose may be administered once a week or several times a week, for example twice or three times per week or every day with doses determined such as to sum up in said weekly dose.
   lamivudine in a dose of between 10 mg and 100 mg per day, for example 10 mg, 20 mg, 30 mg, 40 mg, 50 mg, 60 mg, 70 mg, 80 mg, 90 mg or 100 mg per day, preferably 100 mg per day;
   entecavir in a dose of between 0.1 mg and 10 mg per day, for example 0.1 mg, 0.2 mg, 0.3 mg, 0.4 mg, 0.5 mg, 0.6 mg, 0.7 mg, 0.8 mg, 0.9 mg, 1.0 mg,2.0 mg, 3.0 mg, 4.0 mg, 5.0 mg, 6.0 mg. 7.0 mg, 8.0 mg, 9.0 mg, 10.0 mg per day, preferably 0.5 mg or 1.0 mg per day;
   telbivudine in a dose of between 100 mg and 1000 mg per day, for example 100 mg, 200 mg, 300 mg, 400 mg, 500 mg, 600 mg, 700 mg, 800 mg, 900 mg, or 1000 mg per day, preferably 500 mg, 600 mg, or 700 mg per day;
   administering tenofovir in a dose of between 100 mg and 1000 mg per day, for example 100 mg, 200 mg, 300 mg, 400 mg, 500 mg, 600 mg, 700 mg, 800 mg, 900 mg, or 1000 mg per day, preferably 200 mg, 250 mg, 245 mg, or 300 mg per day;
   administering adefovir is administered in a dose of between 5 mg to 20 mg per day, for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg per day, preferably 10 mg per day; and/or

An inhibitor and a further active ingredient of the invention may be administered sequentially. For example, an inhibitor may be administered for at least one cycle or a course comprising one or more cycles (such as 2, 3, 4, 5, 6, 7, 8, 9 or 10 cycles) followed by the administration of a further active ingredient for at least one cycle or a course comprising one or more cycles (such as 2, 3, 4, 5, 6, 7, 8, 9 or 10 cycles). The duration of one cycle may be 1, 2, 3, 4, 5, 6, 7, 8, 9, 10, 11, or 12 weeks. The duration of one cycle may be at least 1, 2, 3, 4, 5, 6, 7, 8, 9, 10, 11, or 12 weeks. The duration of one course may be 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years.

An inhibitor and a further active ingredient of the invention may be administered concurrently/concomitantly. According to this administration schedule, the administration of an inhibitor timely overlaps with the administration of a further active ingredient. The duration of the administration of an inhibitor and a further active ingredient may be identical or essentially identical. For example, both an inhibitor and a further active ingredient may be administered for at least one cycle or a course comprising one or more cycles (such as 2, 3, 4, 5, 6, 7, 8, 9 or 10 cycles). The duration of one cycle may be 1, 2, 3, 4, 5, 6, 7, 8, 9, 10, 11, or 12 weeks. The duration of one cycle may be at least 1, 2, 3, 4, 5, 6, 7, 8, 9, 10, 11, or 12 weeks. The duration of one course may be 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years. For example, a 24 weeks course of Myrcludes B may be administered at the same time as a 24 weeks course of PEG-IFNa. In this administration scheme, Myrcludex B may be administered daily, whereas PEG-IFNa may be administered weekly. In this administration schedule, an inhibitor and a further active ingredient may be administered simultaneously, for example at essentially the same time or in a single composition.

An inhibitor and a further active ingredient maybe delivered by various delivery routes, depending on the type of ingredient. Administration routs include enteral route (e.g., oraly and rectaly), parenteral route (e.g. intravenously, intramuscularly, subcutaneously intraperitonealy), topically. Preferably, pre-S1 peptide inhibitor is delivered subcutaneously. Preferably, PEG-IFNa is delivered subcutaneously.

The method may be used for human treatment.

### DESCRIPTION OF THE FIGURES

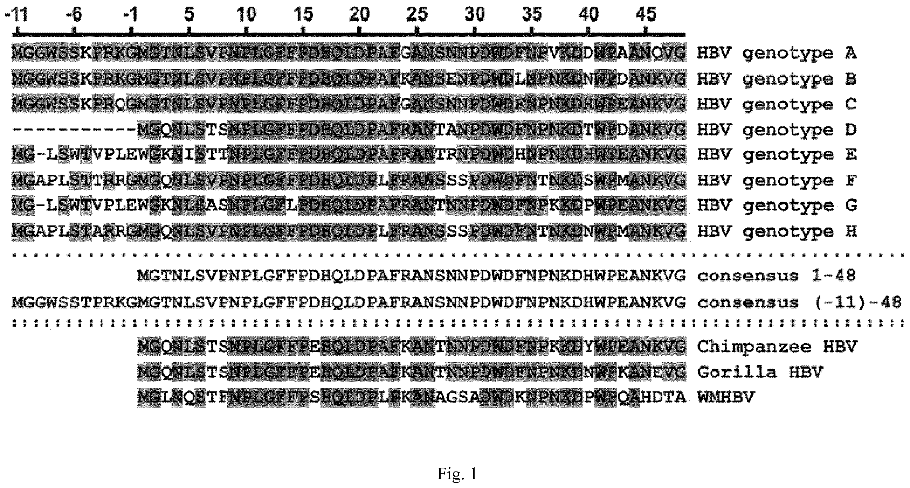

**Figure 1****.** Sequence alignment of the human HBV genotypes A to H and the chimpanzee, gorilla, and woolly monkey hepatitis B viruses. The short (residues 2 to 48) and long (residues -11 to 48) consensus sequences derived from HBV genotypes A to H are depicted in the middle. Identical amino acids are marked by dark-gray boxes. Non-identical amino acids used for the consensus sequences are marked by light-gray boxes.

### DETAILED DESCRIPTION OF THE INVENTION

The invention is based on the finding that a combination of an HBV/HDV pre-S1 entry inhibitor with a nucleotide/nucleoside analogue or an immunomudulator such as interferon results in a decrease or loss of HDV RNA in hepatitis D and/or HBsAg in hepatitis B and D.

In one aspect, the invention relates to combinations and compositions, preferably pharmaceutical compositions, comprising an inhibitor of Na+-taurocholate cotransporting polypeptide (NTCP) and at least one further active ingredient selected from the group comprising a nucleotide analogue, a nucleoside analogue, and an immunomodulator. An NTCP inhibitor may inhibit the HBV/HDV cell entry and may also be referred to as an entry inhibitor.

The combination or compositios may comprise more than one further active ingredient. The compositios may further comprise a pharmaceutically acceptable excipient, carrier etc. The composition may also be referred to as a pharmaceutical composition.

In a combination, the individual active ingredients, i.e., said inhibitor of Na+-taurocholate cotransporting polypeptide (NTCP) and said active ingredient or ingredients are provided in separate vials. In a composition, the individual active ingredients, i.e., said inhibitor of Na+-taurocholate cotransporting polypeptide (NTCP) and said active ingredient are provided in a single vial.

HBV/HDV entry inhibitor of the invention is a compound capable of inhibiting viral entry into a cell such as a hepatocyte. In particular, an HBV/HDV entry inhibitor is capable of binding and inhibiting NTCP receptor on cell surface. Thus, an HBV/HDV entry inhibitor is preferably an NTCP inhibitor.

In one embodiment, an HBV/HDV entry inhibitor may be derived from N-terminal domain of the large (L) HBV surface protein, pre-S1, of any HBV virus, in particular of any HBV strain, genotype or subtype. Examples of HBV strains are HBV strain alpha1, HBV strain LSH (chimpanzee isolate), woodchuck HBV, Woolly Monkey HBV (WMHBV). Examples of HBV subtypes *AD, ADR, ADW, ADYW, AR* and *AYW.* Examples of HBV genotypes are genotypes A to H of human HBV. According to the standard HBV nomenclature, pre-S1 peptide has amino acid coordinates between -11 and 108.

An amino acid sequence alignment of pre-S1 peptides between positions -11 and 48 of various human genotypes as well as respective sequences from pre-S1 peptides of hepatitis B viruses isolated from chimpanzee, gorilla, and woolly monkey is provided in Figure 1. The alignment of the HBV genotypes A to H shows that compared to genotype D, all other genotypes contain an additional 10 (E and G) or 11 (A, B, C, F, and H) N-terminal pre-S1 amino acids. Accordingly, a viral pre-S1 peptide may have different amino acid coordinates and hence a different length, depending on the viral stain, genotype or subtype.

The sequence alignment also indicates that pre-S1 peptides of various genotypes share conserved regions along the peptide length. A highly conserved region is situated between amino acid positions 9 to 21. In addition, individual highly conserved amino acids are situated between amino acids 2 and 6. These conserved sequences are also present in the hepatitis B viruses isolated from chimpanzee, gorilla, and woolly monkey.

Experimental evidence indicate that the capacity of synthetic HBV pre-S I-derived peptides to interfere with the HBV infection depends on the presence of some amino acids within conserved the preS-1 regions (Schulze et al., 2010). Thus, of importance for the peptide activity is a region between amino acid positions 11 and 15 of pre-S1 and in particular amino acids 11, 12, 13, 14 and/or 15 within this region. Further, replacement of some amino acids within a region between positions 2 and 9 are reduced the HBV inhibitory activity of a pre-S1 peptide, whereby amino acid 9 appears essential. Further regions that may contribute to the inhibitory activity of HBV pre-S1-derived peptides are amino acids 2 to 8, 16 to 20, and, to a less pronounced extent, 34 to 48.

Thus, pre-S1 peptide-derived HBV/HDV entry inhibitors (hereinafter referred to as pre-S1 peptide inhibitors) may span amino acids -11 to 108 of HBV pre-S1 (entire HBV pre-S1) or any portion within this region. Hereinafter reference will be made to the standard HBV amino acid numbering. In particular, pre-S1 peptide inhibitor may span amino acids -11 to 78. In some genotypes, the first 10 (genotypes E and G) or 11 (genotypes A, B, C, F, and H) amino acids are absent. Accordingly, pre-S1 peptide inhibitor may span amino acids 1 to 78 of HBV. One or more amino acids beyond amino acid 48, i.e., from amino acid 49 to 108 may be absent. Accordingly, pre-S1 peptide inhibitor may span amino acids -11 to 48. Within the range of -11 to 48, the first 1, 2, 3, 4, 5, 6, 7, 8, 9, 10 or 11 amino acids may be dispensed with. Accordingly, pre-S1 peptide inhibitor may span amino acids 1 to 48. Amino acid 1 (Met) may be absent. Respective pre-S1 peptide inhibitor may span amino acids 2 to 48 and optionally comprise further amino acids at the C-terminus. Within the region of 2 to 48, amino acids 2 to 21 and 34 to 48 are more important for pre-S1 peptide inhibitor function than amino acids 21 to 33. Amino acids 9 to 15, in particular 11 to 15 are most important. Accordingly, pre-S1 peptide inhibitor may consist of amino acids 2 to 48 or a truncated portion thereof. Alternatively, pre-S1 peptide inhibitor may comprise amino acids 2 to 48 or a truncated portion thereof.

A truncated portion may be obtained by amino acid deletions from either end of pre-S1 peptide and have a length of between 46 and 5 amino acids. In particular, a truncated portion may have a length of 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6 or 5 amino acids.

A truncated portion preferably comprises amino acids 9 to 15, 10 to 15, or 11 to 15. A truncated portion preferably consists of amino acids 11 to 15. A truncated portion may gradually be extended by addition of amino acids flanking acids 11 to 15. For example, a truncated portion may consists of amino acids 2 to 15, 3 to 15, 4 to 15, 5 to 15, 6 to 15, 7 to 15, 8 to 15, 9 to 15 or 10 to 15. Any of amino acids 16 to 48 may be used in this example to provide further truncated portions.

In another example, a truncated portion may consist of amino acids 2 to 15, 2 to 16, 2 to 17, 2 to 18, 2 to 19, 2 to 20, 2 to 21, 2 to 22, 2 to 23, 2 to 24, 2 to 25, 2 to 26, 2 to 27, 2 to 28, 2 to 29, 2 to 30, 2 to 31, 2 to 32, 2 to 33, 2 to 34, 2 to 35, 2 to 36, 2 to 37, 2 to 38, 2 to 39, 2 to 40, 2 to 41, 2 to 42, 2 to 43, 2 to 44, 2 to 45, 2 to 45, 2 to 46, 2 to 47 or 2 to 48. Any of amino acids 3 to 11 may be used in this example to provide further truncated portions.

Further examples of truncated portions are amino acids 9 to 15, 2 to 21, 5 to 21, 2 to 15, 2 to 20, 2 to 25, 2 to 30, 2 to 35, 2 to 40.

A truncated portion of pre-S1 peptide may be a functional fragment of pre-S1 peptide retaining the function of pre-S1 peptide in inhibiting HBV cell entry, binding and/or inhibiting NTCT, an essential HBV receptor.

Pre-S1 peptide inhibitors are derived from pre-S1 peptides of various HBV viruses, e.g., various genotypes, strains or subtypes. In particular, pre-S1 peptide inhibitors may be derived from pre-S 1 of genotypes A, B, C, D, E, F, G or H, or of subtypes *AD, ADR, ADW, ADYW, AR andAYW.* Pre-S1 peptides from various HBV viruses will usually be homologues. Homologues from various species have structural and functional similarity and usually share a common evolutionary ancestor. It is envisioned that pre-S1 homologues from further genotypes, strains or subtypes to be identified in the future will be equally suitable as pre-S1 peptide inhibitors.

Pre-S1 peptide inhibitors may be derived from a pre-S1 consensus sequence (Figure 1).

Examples of HBV pre-S1 sequences between amino acids -11 or -10 or 1 (depending on the genotype) and 48 of various genotypes and the consequence sequence are provided below:
HBV preS1 consensus sequence (positions (-11) to 48) 
(-11)-M GGWSS TPRKG MGTNL SVPNP LGFFP DHQLD PAFRA NSNNP DWDFN
PNKDH WPEAN KVG-48 (SEQ ID NO: 1)
HBV preS1 Genotype A sequence (positions (-11) to 48) 
(-11)-M GGWSS KPRKG MGTNL SVPNP LGFFP DHQLD PAFGA NSNNP DWDFN
PVKDD WPAAN QVG-48 (SEQ ID NO: 2)
 HBV preS1 Genotype B sequence (positions (-11) to 48) 
(-11)-M GGWSS KPRKG MGTNL SVPNP LGFFP DHQLD PAFKA NSENP DWDLN
PHKDN WPDAN KVG-48 (SEQ ID NO: 3)
 HBV preS1 Genotype C sequence (positions (-11) to 48) 
(-11)-M GGWSS KPRQG MGTNL SVPNP LGFFP DHQLD PAFGA NSNNP DWDFN
PNKDH WPEAN QVG-48 (SEQ ID NO: 4)
HBV preS1 Genotype D sequence (positions 1 to 48)
1-MGQNL STSNP LGFFP DHQLD PAFRA NTANP DWDFN PNKDT WPDAN KVG-48 (SEQ ID NO: 5)
HBV preS1 Genotype E sequence (positions (-10) to 48)
(-10)-MGLSW TVPLE WGKNI STTNP LGFFP DHQLD PAFRA NTRNP DWDHN PNKDH WTEAN KVG-48 SEQ ID NO: 6)
HBV preS1 Genotype F sequence (positions (-11) to 48) 
(-11)-M GAPLS TTRRG MGQNL SVPNP LGFFP DHQLD PLFRA NSSSP DWDFN
TNKDS WPMAN KVG-48 (SEQ ID NO: 7)
HBV preS1 Genotype G sequence (positions (-10) to 48) 
(-10)-MGLSW TVPLE WGKNL SASNP LGFLP DHQLD PAFRA NTNNP DWDFN
PKKDP WPEAN KVG-48 (SEQ ID NO: 8)
HBV preS1 Genotype H sequence (positions (-11) to 48)

(-11)-M GAPLS TARRG MGQNL SVPNP LGFFP DHQLD PLFRA NSSSP DWDFN
TNKDN WPMAN KVG-48 (SEQ ID NO: 9)

The pre-S1 sequence between amino acids 1 and 48 from Woolly Monkey WMHBV is provided below:
1-MGLNQ STFNP LGFFP SHQLD PLFKA NAGSA DWDKN PNKDP WPQAH DTA (SEQ ID NO: 10)

Pre-S1 peptide inhibitors may contain one or more (e.g., 1, 2, 3, 4, 5, 6, 7,8 9, or 10) amino acid substitutions which do not substantially reduce their HBV inhibitory activity. Preferably, HBV inhibitory activity should not be reduced by more than two orders of magnitude. In particular, reduction by 2-, 3-, 4-, 5-, 6-, 7-, 8-, 9-, 10-, 11-, 12-, 13-, 14-, 15-, 16-, 17-, 18-, 19-, or 20-fold is tolerated. The HBV inhibitory activity may be measured in terms of ICso or IC₉₀. Amino acid substitutions may be conservative or non-conservative. Amino acid substitutions preferably do not affect amino acids important for the HBV inhibitory activity of pre-S1 peptide inhibitors. Individual amino acids located within a highly conserved part of pre-S1 spanning amino acids 2 to 21 are more important for the activity than amino acids outside this region. Within this region, amino acids 9 to 15 are even more important. Among these, amino acids 9, 11, 12 and 13 are most important. Accordingly, amino acid substitutions are preferably located outside amino acids 11 to 15, 9 to 15, 5 to 15, or 2 to 15. Amino acid substitutions may also be located outside amino acids 2 to 8, 16 to 20 or 34 to 48. Amino acid substitutions may be located within amino acids 20 to 23, 26 to 32.

An example of a pre-S1 sequence derived from the pre-S1 sequence of genotype C and containing an amino acid substitution at position 46 (Gln (Q) --> Lys (K)) is provided below:

(-11)-M GGWSS KPRQG MGTNL SVPNP LGFFP DHQLD PAFGA NSNNP DWDFN
PNKDH WPEAN KVG-48 (SEQ ID NO: 11).

A preferred peptide inhibitor is based on pre-S1 peptide between positions 2 and 48 (according to the standard HBV nomenclature) of the amino acid sequence of the HBV pre-S1 consensus sequence:
GTNL SVPNP LGFFP DHQLD PAFRA NSNNP DWDFN PNKDH WPEAN KVG (SEQ ID NO: 12).

Another preferred peptide inhibitor is based on pre-S1 peptide between positions 2 and 48 (according to the standard HBV nomenclature) of the amino acid sequence of the HBV preS1 Genotype C:
GTNL SVPNP LGFFP DHQLD PAFGA NSNNP DWDFN PNKDH WPEAN QVG (SEQ ID NO: 14)

A most preferred peptide inhibitor is based on pre-S1 peptide between positions 2 and 48 (according to the standard HBV nomenclature) of the amino acid sequence of genotype C with an amino acid substitution at position 46 (Gln (Q) → Lys (K))
GTNL SVPNP LGFFP DHQLD PAFGA NSNNP DWDFN PNKDH WPEAN KVG (SEQ ID NO: 13).

A pre-S1 peptide inhibitor may be an N-terminally or C-terminally truncated portion of the above pre-S1 inhibitors of at least 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, or 46 amino acids. The portion retains its functionality as NTCP inhibitor, NTCP binding protein or HBV/HDV entry inhibitor.

Pre-S1 peptide inhibitors may have genotype-spanning inhibitory activity, i.e. they may cross-inhibit genotypes other than the authentic genotype from which the pre-S1 peptide is derived.

Pre-S1 peptide inhibitors are preferably hydrophobic. They may be modified a hydrophobic moiety. Hydrophobic modification by acylation is preferred. Acylation may be acylation with carboxylic acids, fatty acids, or amino acids with lipophilic side chains. Alternatively, pre-S1 peptide inhibitors may be modified by cholesterol, derivatives of cholesterol, phospholipids, glycolipids, glycerol esters, steroids, ceramids, isoprene derivatives, adamantine, farnesol, aliphatic groups, or polyaromatic compounds. Fatty acids may be saturated or unsaturated fatty acids, branched or unbranched fatty acids, preferably with 8 to 22 carbon atoms. Examples of suitable fatty acids for acylation are myristic acid (D14), stearic acid (C18), palmitic acid (C16). Variation of hydrophobic moiety allows for the modulation of specific pre-S1 peptide activities.

A hydrophobic moiety is preferably attached to the N-terminus of the pre-S1 peptide inhibitor. Thus, a hydrophobic moiety may be attached to the N-terminal amino acid of the pre-S 1 peptide inhibitor, or to an amino acid in close proximity to the N-terminus, e.g. amino acids -5, -4, -3, -2, -1, 1, 2, 3, 4, or 5. More than one hydrophobic moiety may be used for the modification of pre-S1 peptide inhibitor. The hydrophobic moieties can be identical or different. The attachment of the hydrophobic moieties is preferably by covalent binding, which can be achieved via carbamate, amide, ether, disulfide or any other linkage that is within the skill of the person skilled in the art.

A preferred peptide inhibitor is a pre-S1 peptide of amino acid sequence
GTNL SVPNP LGFFP DHQLD PAFGA NSNNP DWDFN PNKDH WPEAN KVG (SEQ ID NO: 13),
which is modified at the N-terminus by myristoyl or stearoyl.

The chemical name of the pre-S1 peptide inhibitor Myrcludex B is N-Myristoyl-glycyl-L-threonyl-L-asparaginyl-L-leucyl-L-seryl-L-valyl-L-prolyl-L-asparaginyl-L-prolyl-L-leucyl-glycyl-L-phenylalanyl-L-phenylalanyl-L-prolyl-L-aspartyl-L-histidyl-L-glutaminyl-L-leucyl-L-aspartyl-L-prolyl-L-alanyl-L-phenylalanyl-glycyl-L-alanyl-L-asparaginyl-L-seryl-L-asparaginyl-L-asparaginyl-L-prolyl-L-aspartyl-L-tryptophanyl-L-aspartyl-L-phenylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamide, acetate salt.

An abbreviated chemical name of Myrcludex B is Myristoyl-Gly-Thr-Asn-Leu-Ser-Val-Pro-Asn-Pro-Leu-Gly-Phe-Phe-Pro-Asp-His-Gln-Leu-Asp-Pro-Ala-Phe-Gly-Ala-Asn-Ser-Asn-Asn-Pro-Asp-Trp-Asp-Phe-Asn-Pro-Asn-Lys-Asp-His-Trp-Pro-Glu-Ala-Asn-Lys-Val-Gly-NH2, acetate salt.

Pre-S1 peptide inhibitors may further be modified in a conventional way to improve peptide stability, e.g. stability against degradation. Such modification may include a modification with amide, D-amino acid, modified amino acid, cyclic amino acid, natural polymer, synthetic polymer, or glycine.

In a further embodiment, an HBV/HDV entry inhibitor may be a small molecule.

A further active ingredient of the invention may be an immunomodulator, a nucleotide analogue, or a nucleoside analogue.

Nucleoside analogues are nucleosides which contain a nucleic acid analogue and a sugar. Examples of nucleoside analogues used in the treatment of HBV or HDV are lamivudine, telbivudine and entecavir. Other nucleoside analogues may be used in the practice of the invention.

Nucleotide analogues are nucleotides which contain a nucleic acid analogue, a sugar and one to three phosphate groups. Examples of nucleotide analogues used in the treatment of HBV or HDV are tenofovir and adefovir. Other nucleotide analogues may be used in the practice of the invention.

Example of immunomodulators, i.e. active ingredients that have the ability to modulate the activity of the immune system of a subject, are interferon, therapeutic vaccine, and adjuvant.

Interferon may be interferon alpha, e.g., interferon alpha 2a or interferon alpha 2b. The activity of interferon alpha 2a and interferon alpha 2b may be similar in HBV and HDV infection.

The interferon may be pelylated, i.e. attached to a polyethylene glycol (PEG) moiety. Pegylation may improve pharmacokinetics and convenience for the patient, but does not influence mechanism of action of interferon. In addition, the activity of interferon alpha 2a and 2b is very similar in HBV and HDV infection.

Accordingly, provided are *inter alia* combinations and compositions comprising following ingredients:
a pre-S1 peptide inhibitor such as Myrcludex B and IFNa or PEG-IFNa;
a pre-S1 peptide inhibitor such as Myrcludex B and lamivudine;
a pre-S1 peptide inhibitor such as Myrcludex B and telbivudine;
a pre-S1 peptide inhibitor such as Myrcludex B and entecavir;
a pre-S1 peptide inhibitor such as Myrcludex B and tenofovir; or
a pre-S1 peptide inhibitor such as Myrcludex B and adefovir.
A unit dose of a pre-S1 peptide inhibitor such as Myrcludex B in the combination or
composition may be between 0.5 mg and 20 mg, for example 1 mg, 2 mg, 3 mg, 4, mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, preferably 2 mg, 5 mg, or 10 mg.

A unite dose of interferon or pegylated interferon in the combination or composition may be between 10 µg and 300 µg, for example, 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg, preferably 180 µg.

A unit dose of lamivudine in the combination or composition may be between 10 mg and 100 mg, for example 10 mg, 20 mg, 30 mg, 40 mg, 50 mg, 60 mg, 70 mg, 80 mg, 90 mg or 100 mg, preferably 50 mg or 100 mg.

A unit dose of entecavir in the combination or composition may be between 0.1 mg and 10 mg, for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg, preferably 0.5 mg or 1.0 mg.

A unit dose of telbivudine in the combination or composition may be between 100 mg and 1000 mg, for example 100 mg, 200 mg, 300 mg, 400 mg, 500 mg, 600 mg, 700 mg, 800 mg, 900 mg, or 1000 mg, preferably 500 mg, 600 mg, or 700 mg.

A unite dose of tenofovir in the combination or composition may be between 100 mg and 1000 mg, for example 100 mg, 200 mg, 300 mg, 400 mg, 500 mg, 600 mg, 700 mg, 800 mg, 900 mg, or 1000 mg, preferably 200 mg, 250 mg, 245 mg, or 300 mg.

A unite dose of adefovir in the combination or composition may be between 5 mg to 20 mg, for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg, preferably 10 mg.

An exemplary composition or combination of the invention comprises:
1. 2 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg.
2. 5 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg.
3. 10 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg.
4. 2 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg
5. 5 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg.
6. 10 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg.
7. 2 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg.
8. 5 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg.
9. 10 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg.
10. 2 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg.
11. 5 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg.
12. 10 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg.
13. 2 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg.
14. 5 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg.
15. 10 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg.
16. 2 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg.
17. 5 mg pre-S 1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg.
18. 10 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg.

The above combinations and compositions may be used in the methods of treatment of HBV or HDV infection, hepatitis B or hepatitis D.

In another aspect, the invention relates to the treatment of HBV/HDV infections, hepatitis B and D, and chronic hepatitis B and D by a combination of an NTCP inhibitor / HBV/HDV entry inhibitor and at least one further active ingredient selected from a nucleotide analogue, a nucleoside analogue, and an immunomodulator. This combination therapy leads to a viral RNA decline any eventually results in a decline of HBsAg levels or loss of HBsAg which is a sign of complete cure and hence the ultimate goal of HBV/HDV therapy.

Said inhibitor and said further active ingredient may be provided as a combination or a composition as defined above. More than one further active ingredient may be used in the method.

Thus, the method may, for example, comprise administering to an individual in need of HBV/HDV therapy:
a pre-S 1 peptide inhibitor such as Myrcludex B and IFNa or PEG-IFNa;
a pre-S 1 peptide inhibitor such as Myrcludex B and lamivudine;
a pre-S 1 peptide inhibitor such as Myrcludex B and telbivudine;
a pre-S 1 peptide inhibitor such as Myrcludex B and entecavir;
a pre-S 1 peptide inhibitor such as Myrcludex B and tenofovir; or
a pre-S 1 peptide inhibitor such as Myrcludex B and adefovir.

The method may comprise:
administering a pre-S 1 peptide inhibitor in a dose of between 0.5 an 20 mg per day, for example 1 mg, 2 mg, 3 mg, 4, mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg per day, preferably 2 mg, 5 mg or 10 mg per day for HDV and HBV infection,
and further administering of one or more of following further active ingredients:
   interferon such as pegylated interferon alpha (PEG-IGNa) at the dose of between 10 jug and 300 µg per week, for example, 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg per week, preferably 180 µg per week. A weekly dose may be administered once a week or several times a week, for example twice or three times per week or every day with doses determined such as to sum up in said weekly dose.
   lamivudine at a dose of between 10 mg and 100 mg per day, for example 10 mg, 20 mg, 30 mg, 40 mg, 50 mg, 60 mg, 70 mg, 80 mg, 90 mg or 100 mg per day, preferably 100 mg per day;
   entecavir at a dose of between 0.1 mg and 10 mg per day, for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, 10.0 mg per day, preferably 0.5 mg or 1.0 mg per day;
   telbivudine at a dose of between 100 and 1000 mg per day, for example 100 mg, 200 mg, 300 mg, 400 mg, 500 mg, 600 mg, 700 mg, 800 mg, 900 mg, or 1000 mg per day, preferably 500 mg, 600 mg, or 700 mg per day;
   tenofovir at a dose of between 100 mg and 1000 mg per day, for example 100 mg, 200 mg, 300 mg, 400 mg, 500 mg, 600 mg, 700 mg, 800 mg, 900 mg, or 1000 mg per day, preferably 200 mg, 250 mg, 245 mg, or 300 mg per day;
   adefovir at a dose of between 5 mg to 20 mg per day, for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg per day, preferably 10 mg per day; and/or

The method may comprise administering:
1. 2 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg. The pre-S1 peptide inhibitor and interferon may be administered concurrently. The duration of administration may be 24 weeks or 48 weeks or longer, depending on the progression of the treatment and treatment results.
2. 5 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg. The duration of administration may be 24 weeks or 48 weeks or longer, depending on the progression of the treatment and treatment results.
3. 10 mg pre-S1 peptide inhibitor such as Myrcludex B and 180 µg interferon such as pegylated interferon alpha (PEG-IFNa). The amount of interferon may also vary between 10 µg and 300 µg and be for example 10 µg, 30 µg, 50 µg, 70 µg, 90 µg, 100 µg, 120 µg, 130 µg, 140 µg, 150 µg, 160 µg, 170 µg, 180 µg, 190 µg, 200 µg, 210 µg, 220 µg, 230 µg, 240 µg, 250 µg, 260 µg, 270 µg, 280 µg, 290 µg, or 300 µg. The duration of administration may be 24 weeks or 48 weeks or longer, depending on the progression of the treatment and treatment results.
4. 2 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg. The duration of administration may be 24 weeks or 48 weeks or longer, depending on the progression of the treatment and treatment results.
5. 5 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg. The duration of administration may be 24 weeks or 48 weeks or longer, depending on the progression of the treatment and treatment results.
6. 10 mg pre-S1 peptide inhibitor such as Myrcludex B and 100 mg lamivudine. The amount of lamivudine may also vary between 10 and 100 mg, and be for example 10 mg, 20 mg, 30 mg, 40 mg, 50 mg, 60 mg, 70 mg, 80 mg, 90 mg or 100 mg. The duration of administration may be 24 weeks or 48 weeks or longer, depending on the progression of the treatment and treatment results.
7. 2 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg. The duration of administration may be 24 weeks or 48 weeks or longer, depending on the progression of the treatment and treatment results.
8. 5 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg. The duration of administration may be 24 weeks or 48 weeks or longer, depending on the progression of the treatment and treatment results.
9. 10 mg pre-S1 peptide inhibitor such as Myrcludex B and 0.5 mg or 1.0 mg entecavir. The amount of entecavir may also vary between 0.1 and 10 mg and be for example 0.1 mg, 0.2 mg, 0.3 mg, 0.4 mg, 0.5 mg, 0.6 mg, 0.7 mg, 0.8 mg, 0.9 mg, 1.0 mg, 2.0 mg, 3.0 mg, 4.0 mg, 5.0 mg, 6.0 mg. 7.0 mg, 8.0 mg, 9.0 mg, or 10.0 mg. The duration of administration may be 24 weeks or 48 weeks or longer, depending on the progression of the treatment and treatment results.
10. 2 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
11. 5 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
12. 10 mg pre-S1 peptide inhibitor such as Myrcludex B and 600 mg telbivudine. The amount of telbivudine may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
13. 2 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
14. 5 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
15. 10 mg pre-S1 peptide inhibitor such as Myrcludex B and 200 mg, 245 mg, or 300 mg tenofovir. The amount of tenofovir may also vary between 100 and 1000 mg, and be for example 100 mg, 200 mg, 300 mg, 400 mg, 500 mg, 600 mg, 700 mg, 800 mg, 900 mg, or 1000 mg. The duration of administration may be 24 weeks or 48 weeks or longer, depending on the progression of the treatment and treatment results.
16. 2 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg. The duration of administration may be 24 weeks or 48 weeks or longer, depending on the progression of the treatment and treatment results.
17. 5 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg. The duration of administration may be 24 weeks or 48 weeks or longer, depending on the progression of the treatment and treatment results.
18. 10 mg pre-S1 peptide inhibitor such as Myrcludex B and 100 mg adefovir. The amount of adefovir may also vary between 5 to 20 mg, and be for example 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, or 20 mg. The duration of administration may be 24 weeks or 48 weeks or longer, depending on the progression of the treatment and treatment results.

The duration of administration may be 24 weeks or 48 weeks or longer, depending on the progression of the treatment and side effects.

In the methods of the invention, an inhibitor and a further active ingredient of the invention may be administered sequentially. For example, an inhibitor may be administered for at least one cycle or a course comprising one or more cycles (such as 2, 3, 4, 5, 6, 7, 8, 9 or 10 cycles) followed by the administration of a further active ingredient for at least one cycle or a course comprising one or more cycles (such as 2, 3, 4, 5, 6, 7, 8, 9 or 10 cycles). The duration of one cycle may be 1, 2, 3, 4, 5, 6, 7, 8, 9, 10, 11, or 12 weeks. The duration of one cycle may be at least 1, 2, 3, 4, 5, 6, 7, 8, 9, 10, 11, or 12 weeks. The duration of one course may be 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years or longer.

In the methods of the invention, an inhibitor and a further active ingredient of the invention may be administered concomitantly/concurrently. According to this administration schedule, the administration of an inhibitor timely overlaps with the administration of a further active ingredient. The duration of the administration of an inhibitor and a further active ingredient may be identical or essentially identical. For example, both an inhibitor and a further active ingredient may be administered for at least one cycle or a course comprising one or more cycles (such as 2, 3, 4, 5, 6, 7, 8, 9 or 10 cycles). The duration of one cycle may be 1, 2, 3, 4, 5, 6, 7, 8, 9, 10, 11, or 12 weeks. The duration of one cycle may be at least 1, 2, 3, 4, 5, 6, 7, 8, 9, 10, 11, or 12 weeks. The duration of one course may be 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years. For example, a 24 weeks course of Myrcludes B may be administered at the same time as a 24 weeks course of PEG-IFNa. In this administration scheme, Myrcludex B may be administered daily, whereas PEG-IFNa may be administered weekly. In this administration schedule, an inhibitor and a further active ingredient may be administered simultaneously, for example at essentially the same time or in a single composition.

In the methods of the invention, an inhibitor and a further active ingredient maybe delivered by various delivery routes, depending on the type of ingredient. Administration routs include enteral route (e.g., oraly and rectaly), parenteral route (e.g. intravenously, intramuscularly, subcutaneously intraperitonealy) and topically. Preferably, pre-S1 peptide inhibitor is delivered subcutaneously. Preferably, PEG-IFNa is delivered subcutaneously.

The invention provides various combinations of active ingredients as described above administered at various doses, treatment schedules and routes of administration as described above. For example, the invention provides following treatment regimens:
2 mg Myrcludes B given daily for 24 weeks in combination with 180 µg pegylated interferon alpha (PEG-IFNa) given once a week. The course may be followed by the administration of 2 mg daily Myrcludex B alone for another 24 weeks. Both active ingredients may be delivered subcutaneously. In this example, the daily Myrcludex B dose may also be 5 mg or 10 mg.
2 mg Myrcludes B given daily in combination with a weekly dose of 180 µg pegylated interferon alpha (PEG-IFNa) for. The treatment may last 48 weeks. In this example, the daily Myrcludex B dose may also be 5 mg or 10 mg.
2 mg Myrcludes B given daily in combination with 245 mg tenofovir given daily. The treatment may last for 2 years or longer (indefinitely). In this example, the daily Myrcludex B dose may also be 5 mg or 10 mg.
2 mg Myrcludes B may given daily in combination with 245 mg tenofovir given daily. The treatment may last at least 48 weeks. In this example, the daily Myrcludex B dose may be 5 mg or 10 mg.
2 mg Myrcludes B given daily in combination with 1.0 mg entecavir given daily. The treatment may last for 2 years or longer (indefinitely). In this example, the daily Myrcludex B dose may also be 5 mg or 10 mg.
2 mg Myrcludes B may given daily in combination with 1.0 mg entecavir given daily. The treatment may last at least 48 weeks. In this example, the daily Myrcludex B dose may be 5 mg or 10 mg.

In the methods of the invention, the dose of each active ingredient may be adjusted according to the treatment progression and/or side effects the patient develops during the treatment.

The invention explicitly includes specific combinations or active ingredients and the administration schedule as described in the examples of the application.

The invention will be illustrated by reference to the following non-limiting examples.

### EXAMPLES

### Example 1: Phase 2 clinical trial on Mycludex B in chronic HBV infection

### Objective:

The objective of the clinical trial is the evaluation of safety and tolerability, as well as antiviral efficacy of Myrcludex B in HBsAg-positive patients with active hepatitis.

### Methodology:

Cohort A: 40 chronically HBV infected, HBeAg negative patients (all HBV DNA >2000 IU/ml median HBV DNA 4.7 log₁₀ IU/ml; no cirrhosis) were treated for 12 weeks with once daily sc 0.5mg, 1mg, 2mg, 5mg or 10mg Myrcludex B for 12 weeks (8 patients per dose). Treatment was extended to 24 weeks in patients receiving 10mg.

### Results:

Myrcludex B was very well tolerated, injection side dermatitis occurred in 3 patients receiving 10mg of Myrcludex B, regressed on treatment. Over 1log₁₀ HBV DNA decline at week 12 was observed in 6/8 (75%) patients receiving 10mg Myrcludex B while this occurred less often in the remaining dose groups (7/40; 17%). Alanine transaminase (ALT) normalized in 22/40 (55%) patients and median ALT values declined from 76 U/l before therapy to 36 U/l at week 12 (p<0.001). No significant changes in HBsAg levels became evident.

### Conclusion:

Myrcludex B is a drug candidate for the treatment of chronic hepatitis B (CHB) and chronic hepatitis delta (CHD). Myrcludex B was well tolerated. However, Myrcludex B did not influence the HBsAg levels.

### Example 2: In vitro study of a combination of Myrcludex B and entecavir in HBV/HDV infected cells

A nucleoside analogue entecavir did not improve the inhibition of HBV receptor by Myrcludex B *in vitro* (measured by immunochemistry evaluation of number of HepaRG cells infected with HBV/HDV containing serum):

**Table 1: Number of HBcAg-positive cells on day 6 post infection as determined by HBcAg-specific IF after infection of HepaRG cells with an HDV-positive serum (serum 1) using increasing concentrations of Myrcludex B in the absence of entecavir and in the presence of 20 µM entecavir.**

| Myrcludex B concentration (nM) | No entecavir Mean value (% uncomp. infection) | 20 µM entecavir Mean value (% uncomp. infection) |
|---|---|---|
| 0 | 100 | 100 |
| 0.025 | 44.7 | 36.7 |
| 0.625 | 16.5 | 28.1 |
| 10 | 0.2 | 0.2 |
| 100 | 0 | 0.2 |

Similar results are expected for a combination of Myrcludex B with any nucleoside and nucleotide analogue because they all essentially have the same mechanism of action, i.e., inhibition of HBV polymerase.

### Example 3: In vitro study of a combination of Myrcludex B and IFNa in HBV infected cells

Cell cultures: primary human hepatocytes (PHH), HBV-susceptible cell line HepaRG, cell lines transfected or transduced with HBV receptor NTCP.

Cell cultures will be infected with HBV. Addition of different concentrations of Myrcludex and interferon will be investigated in order to "cure" the cell culture from infection.

### Example 4: Pilot clinical trial with combination therapy of Mycludex B and pegylated IFNa in chronic HDV infection

Myrcludex B did not demonstrate an impact on HBsAg levels *in vivo* (clinical trials with Myrcludex B as monotherapy). The effect of interferon on HBsAg loss in clinical praxis is very modest, with about 3% of patients losing HBsAg after standard 48 weeks therapy. Therefore, it is not obvious that interferon would enhance the impact of Myrcludex B on HBV or HDV infection in terms of reducing HBsAg levels and finally achieving HBsAg loss, i.e., the ultimate goal of HBV therapy.

### Objective:

The objective of the clinical trial is the evaluation of safety and tolerability, as well as antiviral efficacy of Myrcludex B in combination with pegylated interferon alpha (PEG-IFNa) in hepatitis D patients. In particular, effects on virological parameters including HBsAg decline and loss are investigated.

### Methodology:

Cohort B: 24 patients with hepatitis D (compensated liver disease; 12.5% cirrhosis).

Group B 1 ("Myrcludex B monotherapy"): 8 patients scheduled for 24 weeks therapy of 2 mg Myrcludex B daily, delivered subcutaneously.

Group B2: ("PEG-IFNa monotherapy"): 8 patients scheduled for 24 weeks therapy of 180 µg pegylated interferon alpha (PEG-IFNa) weekly, delivered subcutaneously.

Group B3 ("combination therapy"): 8 patients scheduled for 24 weeks therapy of 2 mg Myrcludex B daily, delivered subcutaneously in combination with 180 µg pegylated interferon alpha (PEG-IFNa) weekly, delivered subcutaneously.

Pre-treatment with Myrcludex B before start of interferon treatment, and/or post-treatment with Myrcludex B after end of interferon treatment will also be considered.

### Results:

Myrcludex B was very well tolerated. A psoriasis exacerbation occurred in one HDV patient (B3) leading to discontinuation. In groups B1 and B3 each, one patient discontinued the treatment for various reasons.

Six of the seven patients experienced > 1log10 HDV RNA decline at week 24 during Myrcludex B monotherapy (group B1, Myrcludex B monotherapy) while this response was observed in 7/7 patients of group B2 (PEG-IFNa monotherapy) and group B3 (combination therapy).

HDV RNA became negative in 2/7 patients of groups B1 (Myrcludex B monotherapy) and B2 (PEG-IFNa monotherapy). In contrast, HDV RNA became negative in 5/7 patients (71%) of group B3 (combination therapy).

ALT values declined at week 24 in 6/7 patients (group B1) and 4/7 patients (group B3) and at week 12 in 3/7 patients (group B2,).

One patient (group B3) and 3 patients (group B2) experienced >0.5 log10 HBsAg decline at week 24.

### Conclusion:

Myrcludex B is safe and well tolerated in HBsAg positive patients with or without HDV co-infection. HBV entry inhibition is associated with HBV DNA and HDV RNA declines and improvement of biochemical disease activity. The effect is potentiated by the Myrcludex B/PEG-IFNa combination therapy, wherein complete HDV RNA eradication was observed in over 70% of patients. The reduction in serum HDV RNA is an indication for the reduction of the number of the virus-producing cells when treated with an pre-S1 cell entry inhibitor, without any direct effect on viral replication. In this study, a synergistic effect of pre-S1 peptide inhibitor with interferon was shown on the HDV RNA levels. This effect will translate into a long term viral suppression, also for HBV, and, eventually, lead to viral eradication. Thus, the observed HDV RNA decline and hence virus eradication can be taken as a marker of effective hepatitis D treatment which is expected to project in HBsAg decline and effective hepatitis B treatment with extended treatment duration..

### Example 5: Phase 2 clinical trial with combination therapy of Mycludex B and pegylated IFNa in chronic HBV infection

### Objective:

The objective of the clinical trial is the evaluation of safety and tolerability, as well as antiviral efficacy of Myrcludex B in combination with pegylated interferon alpha (PEG-IFNa) in in patients with chronic HBV infection. In particular, effects on virological parameters including HBsAg decline and loss are investigated.

### Methodology:

Group 1: Pegylated interferon
Group 2: Pegylated interferon with 2 mg Myrcludex B
Group 3: Pegylated interferon in combination with 5 mg Myrcludex B
Group 4: Pegylated interferon in combination with 10 mg Myrcludex B

The treatment with a combination of Myrcludex B and pegylated interferon will be pursued for 24 weeks, followed by 24 week of Myrcludex B monotherapy. The combination will allow increase of the interferon efficacy and the reduction of treatment duration (48 weeks are standard in the treatment with interferons).

### Example 6: Phase 2 clinical trial with combination therapy of Mycludex B and nucleoside/nucleotide analogues in chronic HBV infection

Myrcludex B did not influence HbsAg levels *in vivo* (clinical trials with Myrcludex B as monotherapy). Treatment with nucleoside/nucleotide analogues resulted in HBsAg seroconversion in a very minor proportion of patients (4% of tenofovir patients after two years of treatment and is even lower for the other drugs). Therefore, it is not obvious that nucleoside/nucleotide analogues would enhance the impact of Myrcludex B on HBV or HDV infection in terms of reducing HBsAg levels and finally achieving HBsAg loss, i.e., the ultimate goal of HBV therapy.

### Objective:

The objective of the clinical trial is the evaluation of safety and tolerability, as well as antiviral efficacy of Myrcludex B in combination with a nucleoside analogue (entecavir) or a nucleotide analogue (tenofovir) in patients with chronic HBV infection. In particular, effects on virological parameters including HBsAg decline and loss are investigated.

### Methodology:

Group 1: Nucleoside or nucleotide analogue
Group 2: Nucleoside or nucleotide analogue in combination with 2 mg Myrcludex B
Group 3: Nucleoside or nucleotide analogue in combination with 5 mg Myrcludex B
Group 4: Nucleoside or nucleotide analogue in combination with 10 mg Myrcludex B

The administration of the active ingredients in the above combinations is concurrent.

Separate trials might be performed in treatment-naive patients, and in patients already receiving nucleoside or nucleotide analogue.

Pre-treatment with Myrcludex B before start of nucleoside or nucleotide analogue treatment, and/or post-treatment with Myrcludex B after end of nucleoside or nucleotide analogue treatment will be considered.

### Example 7: Phase 2 clinical trial with combination therapy of Mycludex B and pegylated IFNa in chronic HDV infection

### Objective:

The objective of the clinical trial is the evaluation of safety and tolerability, as well as antiviral efficacy of Myrcludex B in combination with pegylated interferon alpha (PEG-IFNa) in in patients with chronic HDV infection. In particular, effects on virological parameters including HDV RNA loss, HBsAg decline and loss are investigated.

### Methodology:

Group 1: Pegylated interferon
Group 2: Pegylated interferon with 2mg Myrcludex B
Group 3: Pegylated interferon in combination with 5mg Myrcludex B
Group 4: Pegylated interferon in combination with 10mg Myrcludex B

The treatment with a combination of Myrcludex B and pegylated interferon will be pursued for 24 weeks, followed by 24 week of Myrcludex B monotherapy. The combination will allow increase of the interferon efficacy and the reduction of treatment duration (48 weeks are standard in treatment with interferons).

The invention is further described by the following numbered embodiments:
1. A composition or combination comprising an inhibitor of Na+-taurocholate cotransporting polypeptide (NTCP) and a further active ingredient selected from the group consisting of an immunomodulator, a nucleotide analogue, and a nucleoside analogue.
2. The composition or combination of embodiment 1, wherein the NTCP inhibitor is a pre-S1 peptide inhibitor, wherein the pre-S1 peptide inhibitor comprises a pre-S1 peptide of an HBV virus, or a functional fragment thereof, wherein preferably the function is binding to NTCP, inhibition of NTCP, or inhibition of HBV/HDV cell entry.
3. The composition or combination of embodiment 2, wherein the HBV virus is HBV strain alpha1, HBV strain LSH, woodchuck HBV, Woolly Monkey HBV (WMHBV), HBV subtype *AD, ADR, ADW, ADYW, AR* or *AYW,* or HBV genotype A, B, C, D, E, F, G or H.
4. The composition or combination of embodiments 2 or 3, wherein the pre-S1 peptide inhibitor comprises or consists of:
   at least amino acids 2 to 9 and 11 to 15 of a pre-S1 peptide of an HBV virus; or
   at least amino acids 2 to 9 or 11 to 15 of a pre-S 1 peptide of an HBV virus; or
   at least amino acids 9 to 15 of a pre-S1 peptide of an HBV virus; or
   at least amino acids 11 to 15 of a pre-S1 peptide of an HBV virus; or
   amino acids 2 to 48 of a pre-S1 peptide of an HBV virus or a truncated portion thereof of at least 20 amino acids; or
   amino acids 2 to 21 of a pre-S 1 peptide of an HBV virus; or
5. The composition or combination of embodiments 2 to 4, wherein the pre-S1 peptide inhibitor comprises or consists of the amino acid sequence
   GTNL SVPNP LGFFP DHQLD PAFRA NSNNP DWDFN PNKDH WPEAN KVG (SEQ ID NO: 12), or
   GTNL SVPNP LGFFP DHQLD PAFGA NSNNP DWDFN PNKDH WPEAN QVG (SEQ ID NO: 14), or
   GTNL SVPNP LGFFP DHQLD PAFGA NSNNP DWDFN PNKDH WPEAN KVG (SEQ ID NO: 13).
6. The composition or combination of embodiments 2 to 5, wherein the pre-S1 peptide inhibitor is modified by a hydrophobic moiety at the N-terminus, wherein the hydrophobic moiety modification is preferably acylation, wherein the acylation is preferably acylation with myristoyl or stearoyl.
7. The composition or combination of embodiment 6, wherein the pre-S 1 peptide inhibitor is N-Myristoyl-glycyl-L-threonyl-L-asparaginyl-L-leucyl-L-seryl-L-valyl-L-prolyl-L-asparaginyl-L-prolyl-L-leucyl-glycyl-L-phenylalanyl-L-phenylalanyl-L-prolyl-L-aspartyl-L-histidyl-L-glutaminyl-L-leucyl-L-aspartyl-L-prolyl-L-alanyl-L-phenylalanyl-glycyl-L-alanyl-L-asparaginyl-L-seryl-L-asparaginyl-L-asparaginyl-L-prolyl-L-aspartyl-L-tryptophanyl-L-aspartyl-L-phenylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamide, or an acetate salt thereof.
8. The composition or combination of embodiments 2 to 7, wherein a unit dose of pre-S1 peptide inhibitor is between 0.5 mg an 20 mg, preferably 2 mg, 5 mg, or 10 mg.
9. The composition or combination of embodiments 1 to 8, wherein the immunomodulator is an interferon such as interferon alpha, wherein the interferon alpha is preferably interferon alpha 2a or interferon alpha 2b, and wherein the interferon is preferably pelylated.
10. The composition or combination of embodiment 9, wherein a unit dose of interferon is between 10 µg and 300 µg, preferably 180 µg.
11. The composition or combination of embodiments 1 to 10, wherein the nucleotide analogue is tenofovir, or adefovir.
12. The composition or combination of embodiment 11, wherein
   a unit dose of tenofovir is between 100 mg and 1000 mg, preferably 200 mg, 250 mg, 245 mg, or 300 mg; and/or
   a unit dose of adefovir is between 5 mg and 20 mg, preferably 10 mg.
13. The composition or combination of embodiments 1 to 12, wherein the nucleoside analogue is lamivudine, telbivudine, or entecavir.
14. The composition or combination of embodiment 13, wherein
   a unit dose of lamivudine is between 10 mg and 100 mg, preferably 50 mg or 100 mg;
   a unit dose of telbivudine is between 100 mg and 1000 mg, preferably 500 mg, 600 mg, or 700 mg; and/or
   a unit dose of entecavir is between 0.1 mg and 10 mg, preferably 0.5 mg or 1.0 mg.
15. A composition or combination as defined in embodiments 1 to 14 for use in a method of treatment of HBV or HDV infection, hepatitis B, chronic hepatitis B, hepatitis D, or chronic hepatitis D in a subject.
16. The composition or combination for use of embodiment 15, wherein the method comprises administering the pre-S1 peptide inhibitor at a dose of between 0.5 mg and 20 mg per day, preferably 2 mg, 5 mg, or 10 mg per day.
17. The composition or combination for use of embodiment 15 or 16, wherein the method comprises administering:
   interferon, preferably pegylated interferon alpha, at a dose of between 10 µg and 300 µg per week, preferably 180 µg per week; and/or
   lamivudine at a dose of between 10 mg and 100 mg per day, preferably 50 mg or 100 mg per day; and/or
   entecavir at a dose of between 0.1 mg and 10 mg per day, preferably 0.5 or 1.0 mg per day; and/or
   telbivudine at a dose of between 100 mg and 1000 mg per day, preferably 500 mg, 600 mg, or 700 mg per day; and/or
   tenofovir at a dose of between 100 and 1000 mg per day, preferably 200 mg, 245 mg, or 300 mg per day; and/or
   adefovir at a dose of between 5 to 20 mg per day, preferably 10 mg per day.
18. The composition or combination for use of embodiments 15 to 17, wherein the method comprises administering the NTCP inhibitor and/or the further active ingredient 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years or longer.
19. The composition or combination for use of embodiments 15 to 18, wherein the method comprises administering the NTCP inhibitor and/or the further active ingredient parenteraly, preferably intravenously or subcutaneously.
20. The composition or combination for use of embodiments 15 to 19, wherein the subject is a human.
21. A pre-S1 peptide inhibitor as defined in embodiments 2 to 8 for use a method of treatment of HBV or HDV infection, hepatitis B, chronic hepatitis B, hepatitis D, or chronic hepatitis D in a subject, wherein the method comprises the administration of a further active ingredient, wherein the further active ingredient is selected from a group consisting of an immunomodulator, preferably interferon; a nucleotide analogue, preferably tenofovir or adefovir; and an nucleoside analogue, preferably lamivudine, telbivudine and entecavir.
22. The pre-S1 peptide inhibitor for use of embodiment 21, wherein the method is as defined in embodiments 17 to 20.
23. Method of treatment of HBV or HDV infection, hepatitis B, chronic hepatitis B, hepatitis D, or chronic hepatitis D in a subject, comprising administering to the subject the composition or combination of embodiments 1 to 14.
24. The method of embodiment 23, wherein the method comprises administrating to the subject a pre-S1 peptide inhibitor as defined in embodiments 2 to 8 in combination with a further active ingredient, wherein the further active ingredient is selected from a group consisting of an immunomodulator, preferably interferon; a nucleotide analogue, preferably tenofovir or adefovir; and an nucleoside analogue, preferably lamivudine, telbivudine and entecavir.
25. The method of embodiment 23 or 24, wherein the method is as defined in embodiments 17 to 20.

## Claims

1. A pre-S1 peptide inhibitor for use in a method of treatment of HBV or HDV infection, hepatitis B, chronic hepatitis B, hepatitis D, or chronic hepatitis D in a subject, wherein the method comprises administration of further active ingredient, wherein the further active ingredient is an immunomodulator, wherein the immunomodulator is a pegylated interferon alpha, and wherein the pre-S1 peptide inhibitor comprises a pre-S1 peptide of an HBV virus;
wherein the pre-S1 peptide inhibitor is N-Myristoyl-glycyl-L-threonyl-L-asparaginyl-L-leucyl-L-seryl-L-valyl-L-prolyl-L-asparaginyl-L-prolyl-L-leucyl-glycyl-L-phenylalanyl-L-phenylalanyl-L-prolyl-L-aspartyl-L-histidyl-L-glutaminyl-L-leucyl-L-aspartyl-L-prolyl-L-alanyl-L-phenylalanyl-glycyl-L-alanyl-L-asparaginyl-L-seryl-L-asparaginyl-L-asparaginyl-L-prolyl-L-aspartyl-L-tryptophanyl-L-aspartyl-L-phenylalanyl-L-asparaginyl-L-prolyl-L-asparaginyl-L-lysyl-L-aspartyl-L-histidyl-L-tryptophanyl-L-prolyl-L-glutamyl-L-alanyl-L-asparaginyl-L-lysyl-L-valyl-glycinamide.

2. The pre-S1 peptide inhibitor for use of claim 1, wherein the interferon is pegylated interferon alpha 2a or pegylated interferon alpha 2b.

3. The pre-S1 peptide inhibitor for use of claims 1-2, wherein a unit dose of pre-S1 peptide inhibitor is between 0.5 mg and 20 mg, preferably 2 mg, 5 mg, or 10 mg.

4. The pre-S1 peptide inhibitor for use of claims 1-3, wherein a unit dose of interferon is between 10 µg and 300 µg, preferably 180 µg.

5. The pre-S1 peptide inhibitor for use of claims 1-4, wherein the method comprises administering the pre-S1 peptide inhibitor and/or the interferon for 12 weeks, 24 weeks, 36 weeks, 48 weeks, 60 weeks, 1 year, 1.1 years, 1.2 years, 1.3 years, 1.4 years, 1.5 years, 1.6 years, 1.7 years, 1.8 years, 1.8 years, 1.9 years, or 2.0 years, or 3 years, or 4 years or longer.

6. The pre-S1 peptide inhibitor for use of claims 1-5, wherein the method comprises administering the pre-S1 peptide inhibitor and/or the interferon parenterally, preferably intravenously or subcutaneously.

7. The pre-S1 peptide inhibitor for use of claims 1-6, wherein the subject is a human.

8. The pre-S1 peptide inhibitor for use of claims 1-7, wherein the pre-S1 peptide inhibitor and interferon are administered as a combination or composition.

9. The pre-S1 peptide inhibitor for use of claims 1-7, wherein the pre-S1 peptide inhibitor and interferon are administered sequentially.

10. The pre-S1 peptide inhibitor for use of claims 1-7, wherein the pre-S1 peptide inhibitor and interferon are administered concomitantly/concurrently.
